# EUROPEAN PATENT APPLICATION

(11) **EP 2 060 264 A1**
(43) Date of publication of application: **20.05.2009**
(21) Application number: 06849631.4
(22) Date of filing: 27.11.2006
(51) Int. Cl.: A61K 36/45, A61K 31/095, A61K 31/07, A61K 31/51, A61K 31/525, A61K 31/4415, A61K 31/714, A61K 33/30, A61K 9/48, A61P 27/06

(54) **AGENT FOR TREATING EYE DISEASES**

(30) Priority: 29.08.2006 RU 2006131038
(71) Applicant: Otkrytoe Aktsionernoe Obschestvo Zavod Ekologicheskoy Tekhniki I Ekopitaniya 'Diod', Moscow 115114 (RU)
(72) Inventor: TIKHONOV, Vladimir Petrovich, Moscow 113534 (RU); TYUKAVKINA, Nonna Arsenievna, Moscow 119607 (RU)
(74) Representative: Henrion, Oliver
(86) International application number: PCT/RU2006/000629
(87) International publication number: WO 2008/026962

(57) **Abstract**

The invention relates to chemical and pharmaceutical industry, in particular to developing ophthalmological agents having a wide spectrum of action based on vegetable components, vitamins, and trace elements. The agent for treating eye diseases comprises lyophilized blueberries, dihydroquercetin, beta-carotene, selexen, lutein, vitamins B₁, B₂, B₆, and B₁₂, and zinc. The agent having a wide spectrum of action exhibits a reparative, restorative (tonic) and regenerative effect, it does not have any irritating, allergenic, inflammatory or other undesirable effect, and it avoids mechanical contact with the eye surface.

## Description

### Technical field

The invention relates to chemical and pharmaceutical industry, in particular to developing ophthalmological agents having a wide spectrum of action based on vegetable components, vitamins, and trace elements.

### Prior art

Known medical agents for treatment of certain eye diseases are generally used in form of drops or ointments for external application.

Known is an ophthalmological medical agent for treatment of various eye diseases based on an extract of brown algae (Russian patent no. 2104679, A 61 F 9/00).

Further known is a biologically active food additive for treatment and prevention of eye diseases comprising carrot juice, royal jelly, blueberry, ascorbic acid, rutin, vitamin E, nicotinic acid, and sugar, starch, and calcium stearate as adjuvants (Russian patent no. 21097258, A 61 K 35/78).

Known is a phytoconcentrate with therapeutic and preventive activity for patients suffering from diabetes mellitus, the concentrate comprising quercetin, dihydroquercetin, rutin or ascorutin as biologically active ingredient. The concentrate further comprises medical plants and a meat concentrate or a chicken concentrate or a vegetable concentrate. The therapeutic and preventive phytoconcentrate can be used in clinical or ambulant conditions for patients of various age, and it combines prevention and a soft treatment for human beings (Russian patent no. 2123350, A 61 K 35/78).

Further known is a biologically active food additive for prevention of vision disorders and vision correction, the addtive comprising blueberries, a dry gingko biloba extract, a fine powder of dry gingko biloba leaves, magnesium and/or calcium stearate, starch and/or microcristalline cellulose. The additive has a capillary strengthening and antiinflammatory effect on the visual organs, inhibits detrimental effect of free radicals on the visual organs and improves the overall health (Russian patent no. 2270583, A 23 L 1/30).

Known is an agent for increasing visual acuity based on an aqueous decoction of dried Schizandra berries that is either placed in conjunctival sacs for external application or applied to the eye tissue by electrophoresis (Soviet author's certificate no. 171981, A 61 K 35/78).

Known is the use of blueberry leaves and berries for medical treatment.

The use of blueberries has been proved not only to have a broad spectrum of effects on the human organism but also to improve visual acuity, to broaden the visual field and to decrease eye fatigue without action on the eye surface or traumatization of the patient's mind (Gaetan Zheyl, "Extrakt tcherniki i zrenie" ("Blueberry extract and vision"), TASS, Byulleten inostrannoy nautchno-tekhnitcheskoy informatsii TASS, 1964, 72 (962), 1964).

Further it has been established that the flavonoids (rutin, quercetin, vitamin P) that are contained in blueberry strengthen the walls of the blood vessels and regulate their permeability.

Furthermore it is known that preparations with vitamin P are used for treatment of vitamin deficiency, retinal haemorrhage, Schonlein-Henoch disease, and for restoration of neurotrophical processes in the cornea.

The above mentioned medical agents are directed to the improvement of the visual functions. However, their use in form of ointments, films or drops is associated with a mechanical contact with the eye surface and traumatizes it. Besides, each of the known agents shows a specifically directed action.

At the same time there are cases where an excessive use of the eye surface, its irritation and a corresponding psychological traumatization of the patient are highly undesirable, but an action on the visual function is necessary. In such cases medical agents are used that are administered internally *per os* in form of tablets, capsules, teas or extracts.

The technical solution that is closest to the present invention is the biologically active additive (BAA) "Okulist", comprising lyophilized blueberry berries, beta-carotene, dihydroquercetin, and selexen (TU 9197-028-17664661-05 CEZ 3??.99.29.003, July 26, 2005). This additive is recommended in case of retinopathy, night blindness, eye fatigue, glaucoma, and cataract.

Said preparation provides a number of advantages over other known preparations. The internal use avoids traumatization of the eye surface. However, it also shows a directed character of action and does not have a prolonged action.

### Disclosure of the invention

It is an object of the present invention to provide an agent having a wide spectrum of action for treating eye diseases by developing a balanced complex of vegetable components, vitamins, and trace elements.

The technical effect consists in that the agent according to the invention has a reparative, restorative (tonic) and regenerative action, it is ecologically clean and does not show any irritating, allergenic, inflammatory or other undesired effect, it avoids mechanical contact with the eye surface and increases the therapeutic action, and it can also be used for supporting treatment of pathologies of the macula lutea (macular dystrophy) and the retina.

### Best embodiment of the invention

Said object is achieved by the present agent for treating eye diseases comprising lyophilized blueberries, dihydroquercetin, beta-carotene and selexen, and additionally lutein, vitamins B₁, B₂, B₆, and B₁₂, and zinc, with the following amount of the ingredients per capsule, in mg:

| | |
|---|---|
| lyophilized blueberry berries | 282.7 |
| dihydroquercetin | 17.0 |
| beta-carotene | 0.23 |
| selexen | 0.07 |
| lutein | 0.75 - 20.0 |
| vitamin B₁ | 0.255 - 10.2 |
| vitamin B₂ | 0.3 - 12.0 |
| vitamin B₆ | 0.3 - 12.0 |
| vitamin B₁₂ | 0.45 - 18.0 µg |
| zinc | 1.8 - 80.0. |

The agent can be used for improvement of vision in case of retinopathy, night blindness, eye fatigue, glaucoma, and cataract.

The agent can be used as a drug for supporting treatment of pathologies of the macula lutea (macular dystrophy) and the retina.

The agent according to the invention has provisionally been named "Okulist-kompleks".

"Okulist-komplex" is obtained by mixing the ingredients in the indicated amounts and filling the mixture into capsules.

The dosage for prevention of the diseases indicated above is three times a day one capsule during the meal.

The dosage in a supporting therapy is three times a day two capsules during the meal.

Clinical studies on volunteers have been carried out in the Central Military Clinical Hospital of the Ministry of Defense of the Russian Federation, in the Science and Research Institute for Eye Diseases "Gelmgolts" in Moscow, and in the Clinical Pediatric Hospital of the city of Morozovsk.

The results of the clinical studies lead to the following conclusions:
- in none of the cases allergic reactions or undesirable phenomenons were observed during the administration of the drug;
- during the treatment a good tolerability of the drug was observed;
- in comparison to the BAA "Okulist" in case of administration of "Okulist-kompleks" in repeated courses a positive potentiation effect was observed.

The main reason for destruction of the central part of the retina is the action of sunlight, in particular the most aggressive shortwave (UV-, violet and blue) part of its spectrum. The human eye possesses several levels of protection against an excess of solar radiation, namely, the pigmented iris that narrows the pupil, and the macula lutea which is a natural ray filter located directly in front of the most sensitive part of the retina. The colour and light-absorbing properties of the macula are ensured by two natural carotenoids that selectively accumulate in it, namely, lutein and zeaxanthin.

The human organism is not able to synthezise these substances, it is indispensable to provide them from outside.

A solution to this situation would be an increased consumption of vegetables having a high lutein and zeaxanthin content (carrots, spinach, cereals, tomatoes, peas, calendual, etc.), but also the development of pharmaceutical drugs containig these substances.

As no methods of treatment of macular retinopathy are known, at the time being these preventive measures are the only reliable means of fighting age-dependant loss of vision.

Recently biologically active additives appeared that contain lutein obtained from marigold. Studies have confirmed that lutein is well absorbed and stored in the macula lutea, allowing an increase of its density by administration of lutein as an additive (J.A. Mares-Perlman et al., Am. J. Epidemiol.-2001, vol. 62, p. 1448-1461).

Even if lutein is vital with regard to prevention of macular dystrophy, it is also very important to provide the organism, in particular patients belonging to risk groups, with further micronutrients, above all, antioxidants.

### Industrial applicability

The analysis of the data on prevention of eye diseases caused by activation of free radicals and peroxidic processes, namely, cataracts and degeneration of the macula, confirms that the present complex provided a good therapeutical effect. Together with lutein that specifically controls neutralization of singlet oxygen in order to prevent degeneration of the macula, a balanced complex of substances (a vegetable component, vitamins of the B group, beta-carotene, trace elements) supports a normal vision, these substances complementing and increasing each other's effect.

## Claims

1. Agent for treating eye diseases comprising lyophilized blueberry berries, dihydroquercetin, beta-carotene and selexen, **characterized in that** it additionally comprises lutein, vitamins B₁, B₂, B₆, and B₁₂, and zinc, with the following amount of the ingredients per capsule, in mg:
| | |
|---|---|
| lyophilized blueberry berries | 282.7 |
| dihydroquercetin | 17.0 |
| beta-carotene | 0.23 |
| selexen | 0.07 |
| lutein | 0.75 - 20.0 |
| vitamin B₁ | 0.255 - 10.2 |
| vitamin B₂ | 0.3 - 12.0 |
| vitamin B₆ | 0.3 - 12.0 |
| vitamin B₁₂ | 0.45 - 18.0 µg |
| zinc | 1.8 - 80.0. |

2. The agent according to claim 1, **characterized in that** it can be used for improvement of vision in case of retinopathy, night blindness, eye fatigue, glaucoma, and cataract.

3. The agent according to claim 1, **characterized in that** it can be used for supporting treatment of pathologies of the macula lutea (macular dystrophy) and the retina.

4. The agent according to claim 2 or 3, **characterized in that** for prevention of said diseases the dosage is three times a day one capsule during the meal.

5. The agent according to claim 2 or 3, **characterized in that** in a supporting therapy the dosage is three times a day two capsules during the meal.
